# EUROPEAN PATENT APPLICATION

(11) **EP 2 281 510 A1**
(43) Date of publication of application: **09.02.2011**
(21) Application number: 10159229.3
(22) Date of filing: 07.04.2010
(51) Int. Cl.: A61B 8/08

(54) **Providing a plurality of slice images in an ultrasound system**

(30) Priority: 30.07.2009 KR 20090069865
(71) Applicant: MEDISON CO., LTD., Kangwon-do 250-870 (KR)
(72) Inventor: Lee, Kwang Hee, Gangnam-gu, 135-851, Seoul (KR); Kim, Sung Yoon, Gangnam-gu, 135-851, Seoul (KR)
(74) Representative: Schmid, Wolfgang

(57) **Abstract**

Embodiments for providing a plurality of slice images are disclosed. In one embodiment, by way of non-limiting example, an ultrasound system comprises: an ultrasound data acquisition unit configured to transmit and receive ultrasound signals to and from a target object to thereby output ultrasound data; and a processing unit configured to form volume data based on the ultrasound data, the processing unit being configured to set a reference slice, a reference point and a window on the volume data based on input information of a user, the processing unit being configured to set a sagittal view for measuring a thickness of a nuchal translucency (NT) of a fetus on the volume data and set a plurality of slices on the volume data based on the sagittal view, the processing unit being further configured to form a plurality of slice images corresponding to the plurality of slices based on the volume data.

## Description

### CROSS-REFERENCE TO RELATED APPLICATIONS

The present application claims priority from Korean Patent Application No. 10-2009-0069865 filed on July 30, 2009, the entire subject matter of which is incorporated herein by reference.

### TECHNICAL FIELD

The present disclosure generally relates to ultrasound systems, and more particularly to providing a plurality of slice images in an ultrasound system.

### BACKGROUND

An ultrasound system has become an important and popular diagnostic tool since it has a wide range of applications. Specifically, due to its non-invasive and non-destructive nature, the ultrasound system has been extensively used in the medical profession. Modem high-performance ultrasound systems and techniques are commonly used to produce two or three-dimensional diagnostic images of internal features of an object (e.g., human organs).

The ultrasound system transmits and receives ultrasound signals to and from a target object (e.g., a fetus) to thereby form a 2D (two-dimensional) ultrasound image of the fetus. Also, when a user sets a sagittal view for measuring the thickness of a nuchal translucency (NT) of the fetus based on the 2D ultrasound image, the ultrasound system may check a chromosomal abnormality of the fetus by measuring the thickness of the NT based on the sagittal view. However, it may be difficult to precisely set the sagittal view on volume data. Thus, there is a problem in that the thickness of the NT may not be measured exactly.

### SUMMARY

Embodiments for providing a plurality of slice images in an ultrasound system are disclosed herein. In one embodiment, by way of non-limiting example, an ultrasound system comprises: an ultrasound data acquisition unit configured to transmit and receive ultrasound signals to and from a target object to thereby output ultrasound data; and a processing unit placed in communication with the ultrasound data acquisition unit and being configured to form volume data based on the ultrasound data, the processing unit being configured to set a reference slice, a reference point and a window on the volume data based on input information of a user, the processing unit being configured to set a sagittal view for measuring a thickness of a nuchal translucency (NT) of a fetus on the volume data and set a plurality of slices on the volume data based on the sagittal view, the processing unit being further configured to form a plurality of slice images corresponding to the plurality of slices based on the volume data.

In another embodiment, there is provided a method of providing a plurality of slice images, comprising: a) transmitting and receiving ultrasound signals to and from a target object to thereby output ultrasound data; b) forming volume data based on the ultrasound data; c) setting a reference slice, a reference point and a window on the volume data based on input information of a user; d) setting a sagittal view for measuring a thickness of a nuchal translucency (NT) of a fetus on the volume data based on the reference slice, the reference point and the window; e) setting a plurality of slices on the volume data based on the sagittal view; and f) forming a plurality of slice images corresponding to the plurality of slices based on the volume data.

The Summary is provided to introduce a selection of concepts in a simplified form that are further described below in the Detailed Description. This Summary is not intended to identify key or essential features of the claimed subject matter, nor is it intended to be used in determining the scope of the claimed subject matter.

### BRIEF DESCRIPTION OF THE DRAWINGS

- Figure 1: is a block diagram showing an illustrative embodiment of an ultrasound system.
- Figure 2: is a block diagram showing an illustrative embodiment of an ultrasound data acquisition unit.
- Figure 3: is a schematic diagram showing an example of acquiring ultrasound data corresponding to a plurality of frames.
- Figure 4: is a block diagram showing an illustrative embodiment of a processing unit.
- Figure 5: is a schematic diagram showing an example of volume data.
- Figure 6: is a schematic diagram showing an example of a reference slice, a reference point and a window set on the volume data.
- Figure 7: is a schematic diagram showing an example of slices set on the volume data.
- Figure 8: is a schematic diagram showing an example of a slice image, the reference point and the window.

### DETAILED DESCRIPTION

A detailed description may be provided with reference to the accompanying drawings. One of ordinary skill in the art may realize that the following description is illustrative only and is not in any way limiting. Other embodiments of the present invention may readily suggest themselves to such skilled persons having the benefit of this disclosure.

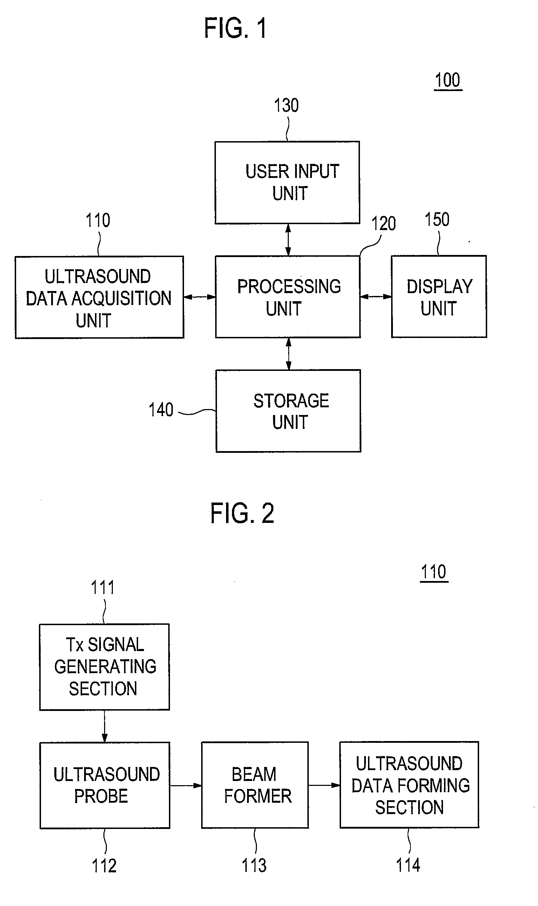

Referring to Figure 1, an ultrasound system 100 in accordance with an illustrative embodiment is shown. As depicted therein, the ultrasound system 100 may include an ultrasound data acquisition unit 110. The ultrasound data acquisition unit 110 may be configured to transmit and receive ultrasound signals to and from a target object (e.g., a fetus) to thereby output ultrasound data. The ultrasound data acquisition unit 110 may include a transmit (Tx) signal generating section 111, as shown in Figure 2.

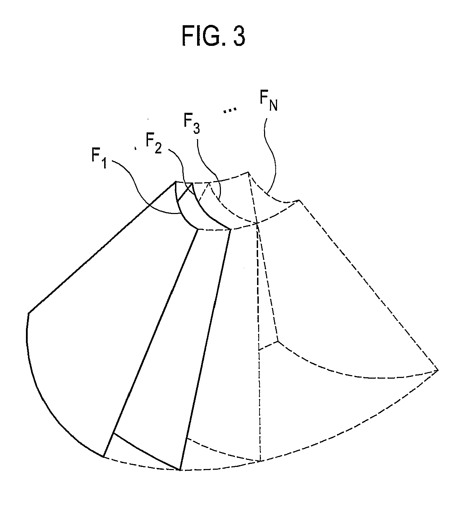

Figure 2 is a block diagram showing an illustrative embodiment of the ultrasound data acquisition unit 110. Referring to Figure 2, the Tx signal generating section 111 may be operable to generate Tx signals. The Tx signal generating section 111 may generate the Tx signals at every predetermined time to thereby form a plurality of Tx signals for obtaining each of frames Fᵢ(1≤ i ≤ N) representing the target object, as shown in Figure 3.

Figure 3 is a schematic diagram showing an example of acquiring ultrasound data corresponding to a plurality of frames Fᵢ(1≤ i ≤ N). The plurality of frames Fᵢ(1≤ i ≤ N) may represent sectional planes of the target object (not shown).

Referring back to Figure 2, the ultrasound data acquisition unit 110 may further include an ultrasound probe 112 containing a plurality of elements for reciprocally converting between ultrasound signals and electrical signals. The ultrasound probe 112 may be configured to transmit ultrasound signals into the target object in response to the Tx signals. The ultrasound probe 112 may further receive echo signals reflected from the target object to thereby output received signals. The received signals may be analog signals. The ultrasound probe 112 may include a three-dimensional mechanical probe, a two-dimensional array probe or the like. However, the ultrasound probe 112 may not be limited thereto.

The ultrasound data acquisition unit 110 may further include a beam former 113. The beam former 113 may be operable to convert the received signals into digital signals. The beam former 113 may further apply delays to the digital signals in consideration of distances between the elements and focal points to thereby output digital receive-focused signals.

The ultrasound data acquisition unit 110 may further include an ultrasound data forming section 114. The ultrasound data forming section 114 may form ultrasound data corresponding to each of the frames Fᵢ(1≤ i ≤ N) based on the digital receive-focused signals. The ultrasound data forming unit 114 may further perform various signal processing (e.g., gain adjustment) to the digital receive-focused signals.

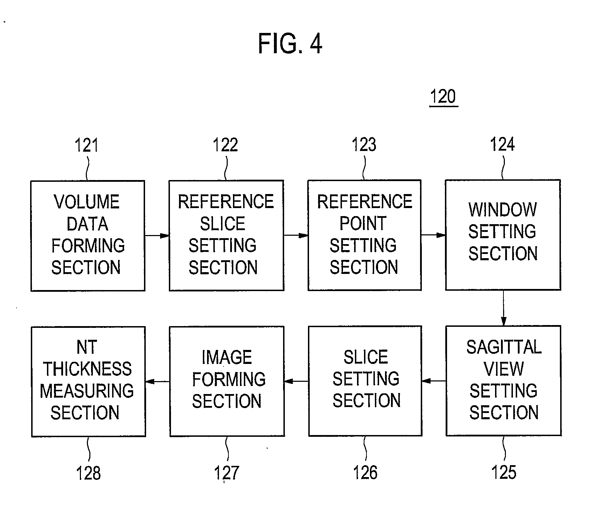

Referring back to Figure 1, the ultrasound system 100 may further include a processing unit 120, which may be coupled to the ultrasound data acquisition unit 110. Figure 4 is a block diagram showing an illustrative embodiment of the processing unit. Referring to Figure 4, the processing unit 120 may include a volume data forming section 121, a reference slice setting section 122, a reference point setting section 123, a window setting section 124, a sagittal view setting section 125, a slice setting section 126, an image forming section 127 and a nuchal translucency (NT) thickness measuring section 128.

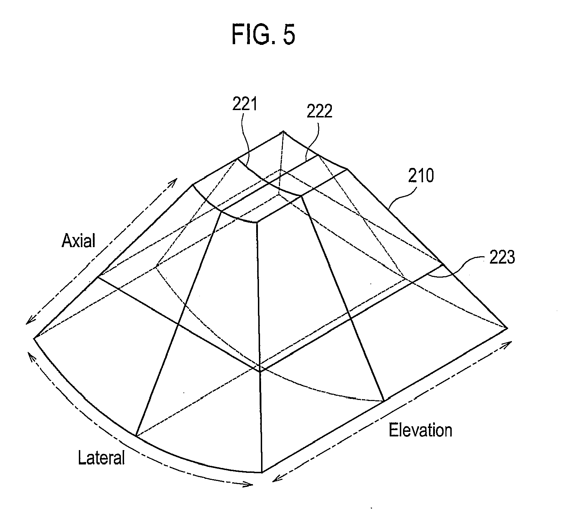

The volume data forming section 121 may synthesize the ultrasound data corresponding to the frames Fᵢ(1≤ i ≤ N) to thereby form volume data 210 including the frames Fᵢ(1≤ i ≤ N), as shown in Figure 5. Figure 5 is a schematic diagram showing an example of volume data 210. The volume data 210 may include a plurality of voxels having brightness values. In Figure 5, reference numerals 221 to 223 represent an A plane, a B plane and a C plane. The A plane 221, the B plane 222 and the C plane 223 are mutually orthogonal. Also, in Figure 5, the axial direction may be a Tx direction of the transducer, the lateral direction may be a longitudinal direction of the transducer, and the elevation direction may be a swing direction of the transducer, i.e., a depth direction of a 3D (three-dimensional) ultrasound image.

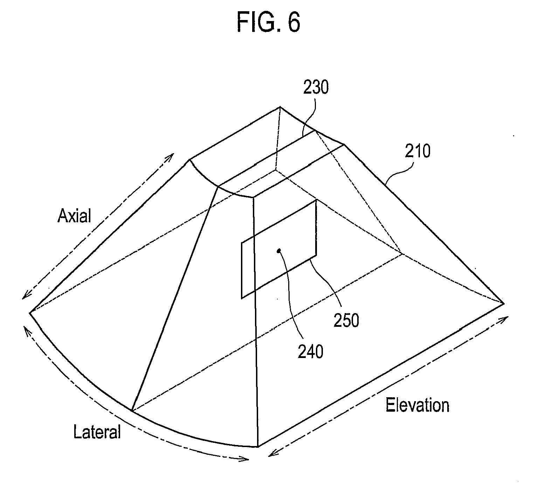

The reference slice setting section 122 may set a reference slice 230 on the volume data 210 based on input information provided from a user input unit 130, as shown in Figure 6. Figure 6 is a schematic diagram showing an example of a reference slice 230, a reference point 240 and a window 250, which are set on the volume data 210. In one embodiment, the reference slice 230 in Figure 6 may be the B plane 222 as shown in Figure 5. However, the reference slice 230 may not be limited thereto.

Referring back to Figure 4, the reference point setting section 123 may set a reference point 240 on the reference slice 230 based on the input information provided from the user input unit 130, as shown in Figure 6.

The window setting section 124 may be operable to set a window 250 to encompass the reference point 240 on the reference slice 230 as shown in Figure 6. In one embodiment, the window 250 may be a rectangular window having a predetermined size. However, the window 250 may not be limited thereto.

The sagittal view setting section 125 may set a sagittal view on the volume data 210 based on the reference slice 230, the reference point 240 and the window 250. The sagittal view may be a slice for measuring a thickness of the NT of the fetus. However, the sagittal view may not be limited thereto.

In one embodiment, the sagittal view setting section 125 may be operable to detect brightness values of pixels within the window 250 set on the reference slice 230. The sagittal view setting section 125 may further calculate a reference value based on the brightness values. The reference value may be a mean value of the brightness values or a sum value of the brightness values. However, the reference value may not be limited thereto. The sagittal view setting section 125 may further move the reference slice 230, the reference point 240 and the window 250 to the lateral direction by predetermined intervals within the volume data 210 to thereby calculate the reference values. Positions of the moved reference slice 230 and the calculated reference values may be stored in a storage unit 140. The sagittal view setting section 125 may further compare the calculated reference values to thereby detect a maximum reference value. The sagittal view setting section 125 may be further operable to move the reference slice 230, the reference point 240 and the window 250 to a position corresponding to the maximum reference value. The sagittal view setting section 125 may also rotate the reference slice 230 and the window 250 to the axial direction by predetermined angles with respect to the reference point 240 to thereby calculate the reference values. Positions of the rotated reference slice 230 and the calculated reference values may be stored in the storage unit 140. The sagittal view setting section 125 may be further operable to compare the calculated reference values to thereby detect a maximum reference value. The sagittal view setting section 125 may further rotate the reference slice 230 and the window 250 to a position corresponding to the maximum reference value. The sagittal view setting section 125 may also rotate the reference slice 230 and the window 250 to the elevation direction by predetermined angles with respect to the reference point 240 to thereby calculate the reference values. The sagittal view setting section 125 may be further operable to compare the calculated reference values to thereby detect a maximum reference value. The sagittal view setting section 125 may rotate the reference slice 230 and the window 250 to a position corresponding to the maximum reference value. Thus, the sagittal view setting section 125 may be operable to set the sagittal view on the volume data 210 based on the reference slice 230. The sagittal view may include the reference point 240 and the window 250. For example, the sagittal view setting section 125 may be operable to set the reference slice 230 as the sagittal view on the volume data 210.

While the sagittal view setting section 125 may set the mean value of the brightness values or the sum value of the brightness values as the reference value in the foregoing embodiment, the sagittal view setting section 125 may further calculate a gradient magnitude and an orientation for each of the pixels within the window 250, form a histogram between the gradient magnitudes and the orientations, detect a peak in the histogram and set the detected peak as the reference value.

Also, while the sagittal view setting section 125 may move the reference slice 230 to the lateral direction and rotate the reference slice 230 to the axial direction and the elevation direction in the foregoing embodiment, the sagittal view setting section 125 may further move and rotate the reference slice 230 to arbitrary directions.

Moreover, while the sagittal view setting section 125 may rotate the reference slice 230 with respect to the reference point 240 in the foregoing embodiment, the sagittal view setting section 125 may further rotate the volume data 210 with respect to the reference point 240.

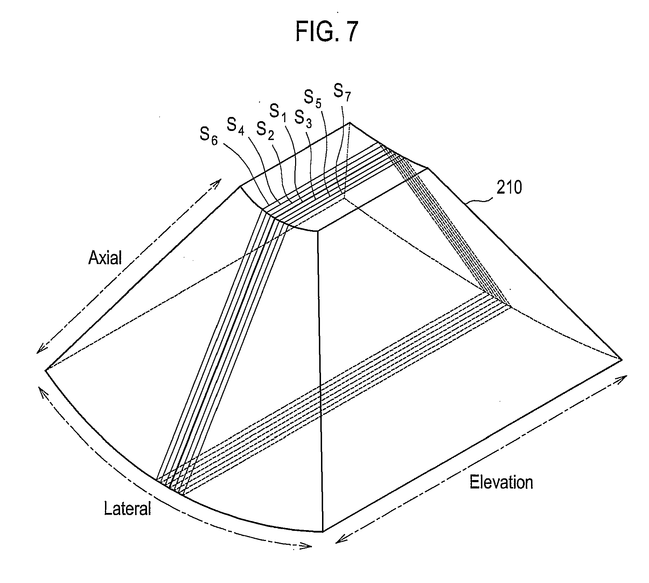

The slice setting section 126 may be operable to set a plurality of slices including the reference point 240 and the window 250 on the volume data 210 with respect to the sagittal view. In one embodiment, the slice setting section 126 may set a plurality of slices S₁ to S₇ including the sagittal view S₁ on the volume data 210 as shown in Figure 7. Figure 7 is a schematic diagram showing an example of slices S₁ to S₇, which are set on the volume data 210. The plurality of slices S₁ to S₇ may have spaces therebetween. However, the plurality of slices S₁ to S₇ may not be limited thereto.

The image forming section 127 may form a plurality of slice images (not shown) corresponding to the plurality of slices S₁ to S₇ based on the volume data 210. The slice images may be a brightness mode image. The image forming section 127 may further render the volume data 210 to thereby form a 3D (three-dimensional) ultrasound image (not shown).

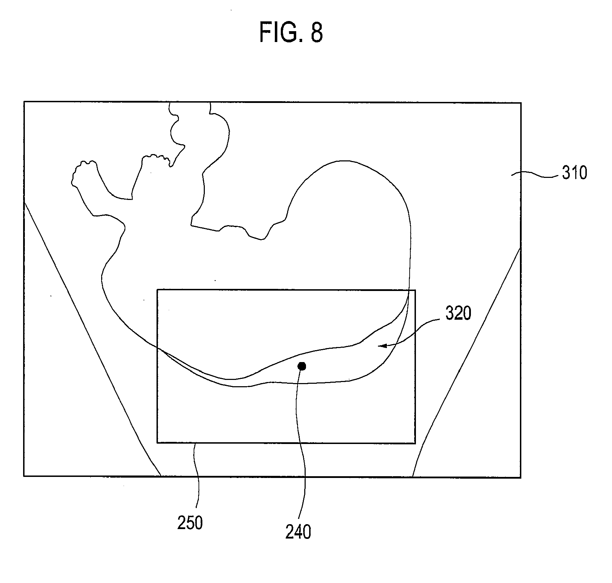

The NT thickness measuring section 128 may extract at least one of slice image corresponding to the input information provided from the user input unit 130 from the plurality of slice images. The NT thickness measuring section 128 may further set the reference point 240 and the window 250 on the extracted slice image 310 as shown in Figure 8. Figure 8 is a schematic diagram showing an example of a slice image 310, the reference point 240 and the window 250. The NT thickness measuring section 128 may be further operable to detect a contour of the NT 320 within the window 250. The contour may be detected by using an edge mask such as Sobel, Prewitt, Robert, Canny mask or the like. The contour may be detected based on the differences between eigenvalues using structure tensors. The NT thickness measuring section 128 may further measure a thickness of the NT based on the detected contour to thereby output measurement information. The methods of measuring the thickness of the NT thickness based on the contour are well known in the art. Thus, they have not been described in detail so as not to unnecessarily obscure the present invention.

Referring back to Figure 1, the ultrasound system 100 may further include the user input unit 130. The user input unit 130 may be operable to receive input, information of a user. In one embodiment, the input information may include first input information for setting the reference slice on the volume data, second input information for setting the reference point on the NT of the reference slice and third input information for selecting at least one of the slice images from the plurality of slice images. The user input unit 130 may include a control panel, a mouse, a keyboard or the like. However, the user input unit 130 may not be limited thereto.

The ultrasound system 100 may further include the storage unit 140. The storage unit 140 may store the positions of the reference slice 230 and the reference values. The storage unit 140 may further store the volume data 210.

The ultrasound system may further include a display unit 150. The display unit 150 may display the plurality of slice images, the extracted slice image, the measurement information and the three-dimensional ultrasound image.

Although embodiments have been described with reference to a number of illustrative embodiments thereof, it should be understood that numerous other modifications and embodiments can be devised by those skilled in the art that will fall within the spirit and scope of the principles of this disclosure. More particularly, numerous variations and modifications are possible in the component parts and/or arrangements of the subject combination arrangement within the scope of the disclosure, the drawings and the appended claims. In addition to variations and modifications in the component parts and/or arrangements, alternative uses will also be apparent to those skilled in the art.

## Claims

1. An ultrasound system, comprising:
an ultrasound data acquisition unit configured to transmit and receive ultrasound signals to and from a target object to thereby output ultrasound data; and
a processing unit placed in communication with the ultrasound data acquisition unit and being configured to form volume data based on the ultrasound data, the processing unit being configured to set a reference slice, a reference point and a window on the volume data based on input information of a user, the processing unit being configured to set a sagittal view for measuring a thickness of a nuchal translucency (NT) of a fetus on the volume data and set a plurality of slices on the volume data based on the sagittal view,
the processing unit being further configured to form a plurality of slice images corresponding to the plurality of slices based on the volume data.

2. The ultrasound system of Claim 1, further comprising a user input unit placed in communication with the processing unit and being configured to receive the input information.

3. The ultrasound system of Claim 1, wherein the input information comprises:
a first input information for setting the reference slice on the volume data; and
a second input information for setting the reference point on the reference slice.

4. The ultrasound system of Claim 1, wherein the processing unit comprises:
a volume data forming section configured to form the volume data based on the ultrasound data;
a reference slice setting section configured to set the reference slice on the volume data based on the input information;
a reference point setting section configured to set the reference point on the reference slice based on the input information;
a window setting section configured to set the window to encompass the reference point on the reference slice;
a sagittal view setting section configured to set the sagittal view on the volume data based on the reference slice, the reference point and the window;
a slice setting section configured to set the plurality of slice including the sagittal view on the volume data; and
an image forming section configured to form the plurality of slice images corresponding to the plurality of slices based on the volume data.

5. The ultrasound system of Claim 4, wherein the sagittal view setting section is configured to:
move the reference slice, the reference point and the window to a first direction by predetermined intervals within the volume data to thereby calculate first reference values;
detect a maximum first reference value from the first reference values;
move the reference slice, the reference point and the window to a position corresponding to the maximum first reference value;
rotate the reference slice and the window by predetermined angles with respect to the reference point to thereby calculate second reference values;
detect a maximum second reference value from the second reference values; and
rotate the reference slice and the window to a position corresponding to the maximum second reference value to thereby set the sagittal view on the volume data.

6. The ultrasound system of Claim 5, wherein the sagittal view setting section is configured to detect brightness values of pixels within the window, calculate a mean value of the brightness values, and set the mean value as the first or second reference value.

7. The ultrasound system of Claim 5, wherein the sagittal view setting section is configured to detect brightness values of pixels within the window, calculate a sum value of the brightness values, and set the sum value as the first or second reference value.

8. The ultrasound system of Claim 5, wherein the sagittal view setting section is configured to calculate a gradient magnitude and an orientation for each of pixels within the window, form a histogram between the gradient magnitude and the orientation, detect a peak in the histogram, and set the detected peak as the first or second reference value.

9. The ultrasound system of Claim 4, wherein the processing unit further comprises a NT thickness measuring section configured to extract at least one of slice image corresponding to the input information from the plurality of slice images, detect a contour of the NT within the window at the extracted slice image, and measure a thickness of the NT based on the detected contour.

10. A method of providing a plurality of slice images, comprising:
a) transmitting and receiving ultrasound signals to and from a target object to thereby output ultrasound data;
b) forming volume data based on the ultrasound data;
c) setting a reference slice, a reference point and a window on the volume data based on input information of a user;
d) setting a sagittal view for measuring thickness of a nuchal translucency (NT) of a fetus on the volume data based on the reference slice, the reference point and the window;
e) setting a plurality of slices on the volume data based on the sagittal view; and
f) forming a plurality of slice images corresponding to the plurality of slices based on the volume data.

11. The method of Claim 10, wherein the input information comprises first input information for setting the reference slice on the volume data and second input information for setting the reference point on the reference slice.

12. The method of Claim 10, wherein the step c) comprises:
setting the reference slice on the volume data based on the input information;
setting the reference point on the reference slice based on the input information; and
setting the window to encompass the reference point on the reference slice.

13. The method of Claim 11, wherein the step d) comprises:
d1) moving the reference slice, the reference point and the window to a first direction by predetermined intervals within the volume data to thereby calculate first reference values;
d2) detecting a maximum first reference value from the first reference values;
d3) moving the reference slice, the reference point and the window to a position corresponding to the maximum first reference value;
d4) rotating the reference slice and the window to a second direction by predetermined angles with respect to the reference point to thereby calculate second reference values;
d5) detecting a maximum second reference value from the second reference values; and
d6) rotating the reference slice and the window to a position corresponding to the maximum second reference value to thereby set the sagittal view on the volume data.

14. The method of Claim 13, wherein the first or second reference value is set based on one of a mean value of brightness values of pixels within the window, a sum value of brightness values of pixels within the window and a peak in a histogram between a gradient magnitude and an orientation for each of pixels within the window.

15. The method of Claim 11, further comprising the steps of:
g) receiving third input information for selecting at least one of slice image from the plurality of slice images;
h) extracting the at least one of slice image from the plurality of slice images based on the third input information;
i) detecting a contour of the NT within the window at the extracted slice image; and
j) measuring a thickness of the NT based on the detected contour.
